# EUROPEAN PATENT SPECIFICATION

(11) **EP 0 381 750 B1**
(45) Date of publication and mention of the grant of the patent: **30.11.1994**
(21) Application number: 89910341.0
(22) Date of filing: 02.08.1989
(51) Int. Cl.: A61K 31/59

(54) **METHOD FOR TREATING AND PREVENTING LOSS OF BONE MASS**
VERFAHREN ZUR BEHANDLUNG UND VORBEUGUNG DES VERLUSTES VON KNOCHENMASSE
PROCEDE DE TRAITEMENT ET DE PREVENTION DE LA PERTE DE MASSE OSSEUSE

(30) Priority: 02.08.1988 US 227371
(43) Date of publication of application: 16.08.1990
(73) Proprietor: BONE CARE INTERNATIONAL, INC., Madison, WI 53713 (US)
(72) Inventor: DeLUCA, Hector, F., Deerfield, WI 53531 (US); BISHOP, Charles, W., Madison, WI 53713 (US); MAZESS, Richard, B., Madison, WI 53711 (US); GALLAGHER, John, C., Omaha, NE 68131 (US)
(74) Representative: Ford, Michael Frederick
(86) International application number: US8903341
(87) International publication number: WO9001321

(56) References cited:
- EP-A- 0 197 514
- WO-A-84/04527
- US-A- 4 225 596
- US-A- 4 588 716
- US-A- 5 104 864
- Disorders of Bone and Mineral Metabolism; 1992 Raven Press, New York USA, pages 831, 856-873
- Bone and Mineral, 1992, vol. 18, p.227-236
- Nephron 44, p.161-166, 1986
- Biochem.J. (1982), vol. 204, p.185-189
- Analytical Biochemistry, vol. 116, p.189-203 (1981)
- J.Clin. Endocrin.Metab. 1985, vol. 60, p.615-617
- Calcif. Tissue Int. (1986), vol. 38, p.328-332
- Acta Med. Scand. 1985, vol. 218, p.329-33
- The Lancet, 19.05.1984, p.1091-93
- The American Journal of Medicine, 1988, vol. 84, p.401-408
- Endocrinol.Japan. 1985, vol32(2), p.305-315
- Acta Med. Scand. 1980, vol. 207, p.221-224
- Arch. Biochem. Biophys., volume 186, no. 1, February 1978, Academic Press, Inc., L.E. Reeve et al., pages 164-167
- Proc. Soc. Exp. Biol. Med., volume 178, 1985, Society for Experimental Biology and Medicine, G. Sjöden et. al. pages 432-436
- J. Nutr., volume 114, 1984, American Institute of Nutrition, G. Sjöden et al.; pages 3043-2046
- Science, volume 186, no. 4168, 1974, H.Y.P Lam; pages 1038-1040
- Acta Endocrinol. (Copenh.), volume 16, no.4, August 1984, G.0.J. Sjöden et al.; pages 564-568

## Description

This invention relates to a method for treating and preventing metabolic bone disorders characterized by loss of bone mass or by disproportionately low bone mineral content.

More specifically, this invention relates to a method for treating or preventing various known forms of osteoporosis, e.g. postmenopausal, senile and steroid-induced osteoporosis or other disease states one of the characteristics of which is the loss of bone mass or decreased mineral content.

Still more specifically, this invention relates to a method for treating or for preventing the depletion of bone of women who are entering menopause or who are postmenopausal.

Numerous metabolic bone disorders are known to the medical community which are characterized by loss of bone mass or disproportionate mineralization of bone. These disorders include postmenopausal osteoporosis, senile osteoporosis, renal osteodystrophy, corticosteroid-induced osteopenia, and anticonvulsant osteomalacia. Of these disorders, postmenopausal and senile osteoporosis are most commonly encountered in normal medical practice.

As a group, these bone depletive disorders are a major and growing public health problem in the United States. Together, they cause more than 1 million bone fractures per year, primarily of the spine, hip, and distal forearm, and result in an annual cost of $6 to 7 billion to the American society. Unfortunately, the incidence of these bone disorders will rise significantly as the mean age of the U.S. population continues to increase.

Despite differing etiologies, the aforementioned metabolic bone disorders develop during an extended period of negative calcium balance. This commonality of the disorders suggests that agents which stimulate intestinal calcium absorption may be effective in restoring calcium balance and thereby treating or preventing the development of such bone disorders. It has long been known that Vitamin D plays a critical role in stimulating calcium absorption and regulating calcium metabolism. The discovery of the active forms of Vitamin D, [M.F. Holick et al., Proc. Natl. Acad. Sci. USA 68, 803-804 (1971); G. Jones et al., Biochemistry 14, 1250-1256 (1975)] and active Vitamin D analogues [M. F. Holick et al., Science 180, 190,191 (1973); H. Y. Lam et al., Science 186, 1038-1040 (1974)], caused much excitement and speculation about the usefulness of these compounds in the treatment of bone depletive disorders.

Animal studies examining the effects of these active Vitamin D compounds suggested that such agents would be useful in restoring calcium balance. Further, an early clinical study indicated that administration of 0.5 µg/day of 1,25-dihydrorycholecalciferol (1,25 Vitamin D₃) to a group of postmenopausal women improved the intestinal calcium absorption as well as the calcium balance of these women. On this basis, U.S. Patent 4,225,596 (" '596 Patent") described and claimed the use of 1,25 Vitamin D₃ for increasing calcium absorption and retention. Such use also was claimed in the same patent for 1,25 dihydroxyergocalciferol (1,25 Vitamin D₂) and 1α-hydroxyergocalciferol (1α-Vitamin D₂), which the patent teaches are "eminently suitable and readily substitutable for the 1,25 dihydroxycholecalciferol."

The best indicator of the efficacy of Vitamin D compounds to prevent or treat depletive bone disorders is bone itself rather than calcium absorption or calcium balance. More recent clinical data indicates that at the dosage ranges taught in the '596 Patent, 1,25 Vitamin D₃ has, at best, modest efficacy in preventing or restoring loss of bone mass or bone mineral content [S.M. Ott, C.H. Chesnut, In: J Jensen et al, ed, Norhaven A/S, Viborg, p.83 (1987); J.C. Gallagher et al., 7th Workshop on Vitamin D, Rancho Mirage, p. 196 (1988); J. Aloia et al., Amer. J. Med. 84:401-08 (1988)].

Together these clinical studies with 1,25 Vitamin D₃, and one other conducted with 1α-Vitamin D₃ [M. Shiraki et al., Endocrinol. Japan 32, 305-315 (1985)], indicate that the ability of these agents to restore lost bone mass or bone mineral content is dose related. These studies also indicate, however, that at the dosage ranges required for the agents to be truly effective, toxicity in the form of hypercalcemia and hypercalciuria becomes a major problem. Thus, attempts to increase the amount of 1,25 Vitamin D₃ above 0.5 µg/day have frequently resulted in toxicity. At dosage levels below 0.5 µg/day no effects are observed on bone. [See G.F. Jensen et al., Clin. Enocrinol. 16, 515-524 (1982); C. Christiansen et al., Eur. J. Clin. Invest. 11, 305-309 (1981)]. Two µg/day of 1α-Vitamin D₃ was found to have efficacy in increasing bone mass in patients exhibiting senile osteoporosis [O.H. Sorensen et al., Clin. Endocrinol. 7, 169S-175S (1977)]. Data from the clinical studies in Japan, a population that has low calcium intake, indicate that efficacy is found with 1α-Vitamin D₃ when administered at 1 µg/day [M. Shiraki et al., Endocrinol. Japan. 32:305-315 (1985); H. Orimo et al., Bone and Mineral 3, 47-52 (1987)]. At 2 µg/day, however, toxicity with 1α-Vitamin D₃ occur in approximately 67 percent of the patients, and at 1 µg/day this percentage is approximately 20 percent.

Thus, the prior art teaches that due to their toxicity, 1 hydroxylated Vitamin D compounds can only be administered at dosages that are, at best, modestly beneficial in preventing or treating loss of bone or bone mineral content. Indeed, Aloia recommends that alternative routes of administration be sought which might avoid the toxicity problems and allow higher dosage levels to be achieved. [J.Aloia et al, Amer J Med 84:401-408 (1988)].
EP-A-197514 teaches that bone mass may be increased by administration of a parathyroid hormone or physiologically active fragment thereof, in combination with a hydroxylated vitamin D compound or a non-toxic calcium salt. Possible dose ranges are quoted and broad limits for 1-alpha-hydroxy vitamin D₃ and 1-alpha-hydroxy vitamin D₂ (which are not distinguished from each other) are stated as 0.05 to 3.0 µg per day.

Hitherto, no clinical data were available with respect to the 1-hydroxylated Vitamin D₂ compound. Nonetheless, in the prior art, that compound has been considered as essentially equivalent to its Vitamin D₃ counterpart. Thus, when the prior art teaches that because of its extreme toxicity 1α-Vitamin D₃ may not be safely administered at normal calcium intakes in excess of 2 µg/day, a person skilled in the art would understand that the same warning also applies to 1α-Vitamin D₂.

During the course of our investigations, we have compared 1α-Vitamin D₂ to 1α-Vitamin D₃. 1α-Vitamin D₂ is equally active as 1α-Vitamin D₃ in the healing of rickets, the stimulation of intestinal calcium absorption and in the elevation of serum inorganic phosphorous of rachitic rats. [G. Sjoden et al., J. Nutr. 114, 2043-2946 (1984)]. In the same laboratory animal we also have found that, 1α-Vitamin D₂ is 5 to 15 times less toxic than 1α-Vitamin D₃. [G. Sjoden et al., Proc. Soc. Exp. Biol. Med. 178, 432-436 (1985)].

It has now been found that 1α-Vitamin D₂ may be safely administered to human subjects experiencing or having a tendency toward loss of bone mass or bone mineral content at dosages greater than 3 µg/day.

According to a first aspect of this invention there is provided the use of 1-alpha-hydroxy vitamin D₂, for the preparation of a medicament for the treatment or prevention of human osteoporosis or osteopenia by the administration of at least 2.0 µg per day of the 1-alpha-hydroxy vitamin D₂, wherein the medicament is free of parathyroid hormone and free of active fragments thereof.

In a second aspect this invention provides a pharmaceutical composition which is a single dose in the form of a tablet, lozenge or capsule which contain at least 2.0 µg of 1-alpha-hydroxy vitamin D₂, also contains a pharmaceutically acceptable excipient, and is free from parathyroid hormone and free of active fragments thereof.

Those bone depletive disorders classified as osteoporosis, for which the present invention is intended to be used are particularly post-menopausal osteoporosis, senile osteoporosis, idiopathic osteoporosis, immobilization osteoporosis, post-lactational osteoporosis, glucocorticoid, alcohol or drug-induced osteoporosis, juvenile osteoporosis, osteoporosis secondary to gonadal insufficiency, malnutrition, hyperprolactinemia, or disorders of the gastrointestinal tract, liver, or kidneys, and osteoporosis that is a sequela of prior osteomalacia, chronic disorders involving the bone marrow, and heritable forms of osteoporosis such as osteoporosis imperfecta and its variants, and other heritable disorders of connective tissue.

In a third aspect this invention provides the use of 1-alpha-hydroxy vitamin D₂, for the preparation of a medicament for the reversal of human osteoporosis by the administration of at least 2.0 µg per day of the 1-alpha-hydroxy vitamin D₂, wherein the medicament is free of parathyroid hormone and free of active fragments thereof.

### Example 1

Six postmenopausal osteoporotic women were enrolled in an open label study. The selected patients had ages between 55 and 75 years, and exhibited L2-L3 vertebral bone mineral density between 0.7 and 1.05 g/cm², as determined by measurements with a LUNAR Radiation dual photon absorptiometer. (The mean bone mineral density in women with osteoporosis is about 0.85 ± 0.17 g/cm², so that these limits correspond to about the 15th to the 85th percentiles).

On admission to the study, all patients received instruction on selecting a daily diet containing 400 to 600 mg. of calcium. Compliance to this diet was verified at weekly intervals by 24-hour food records and by interviews with each patient.

All patients completed a one-week baseline period, a five-week treatment period, and a one-week post-treatment observation period. During the treatment period, patients orally self-administered a 1α-Vitamin D₂ at an initial dose of 0.5 µg/day for the first week, and at successively higher doses of 1.0, 2.0, 4.0 and 5.0 µg/day in each of the following four weeks. All doses were administered before breakfast.

Blood and urine chemistries were monitored on a weekly basis throughout the study. Key blood chemistries included fasting serum levels of calcium, phosphorus, osteocalcin, creatinine and blood urea nitrogen. Key urine chemistries included 24-hour excretion of calcium, phosphorus and creatinine.

Data from the study clearly demonstrated that 1α-Vitamin D₂ can be safely administrated at high dose levels. As shown in Table 1, all six patients tolerated a 4.0 µg/day dose of this compound without exhibiting hypercalciuria (>350 mg/24 hr) or hypercalcemia (>11.0 mg/dL). Five of the patients tolerated a 5.0 µg/day dosage without side effects, while the sixth patient showed a mild hypercalciuria, without attendant hypercalcemia, at this dosage.

**TABLE 1**

| Dose-response relationship between orally administered 1α-Vitamin D₂ and fasting serum calcium and 24-hour urinary calcium values in postmenopausal osteoporotic patients. | | | | |
|---|---|---|---|---|
| Dose | Fasting Serum Ca | | 24-hr Urinary Ca | |
| | Mean ± SE | Range | Mean ± SE | Range |
| 0.5 µg/d | 9.80 ± .10 | (9.51-10.20) | 121.8 ± 17.2 | (55.3-180.0) |
| 1.0 µg/d | 9.81 ± .08 | (9.61-10.18) | 132.2 ± 16.7 | (67.8-187.1) |
| 2.0 µg/d | 9.87 ± .15 | (9.58-10.56) | 169.9 ± 16.4 | (110.5-215.1) |
| 4.0 µg/d | 9.92 ± .10 | (9.65-10.33) | 193.9 ± 30.1 | (133.8-324.1) |
| 5.0 µg/d | 9.80 ± .09 | (9.61-10.15) | 221.3 ± 37.0 | (149.9-405.9) |

Additional blood and urine data from this clinical study supported the conclusion that 1α-Vitamin D₂ can be safely administered at high dosages. In particular, this compound did not adversely affect kidney function, as determined by creatinine clearance and blood levels of urea nitrogen; nor did it increase urinary excretion of hydroxyproline, indicating the absence of any stimulatory effect on bone resorption. The compound had no effect on any routinely monitored serum parameters, indicating the absence of adverse metabolic effects.

A positive effect of 1α-Vitamin D₂ on calcium homeostasis was evident from modest increases in 24-hour urinary calcium levels (see Table 1), confirming that the compound increases intestinal calcium absorption.

### Example 2

A clinical study is conducted with postmenopausal osteoporotic out-patients having ages between 55 and 75 years. The study involves up to 120 patients randomly divided into three treatment groups, and continues for 12 to 18 months. Two of the treatment groups receive constant dosages of 1α-Vitamin D₂ (u.i.d.; two different dose levels above 3.0 µg/day) and the other group receives a matching placebo. All patients maintain a normal intake of dietary calcium (500 to 800 mg/day) and refrain from using calcium supplements. Efficacy is evaluated by pre- and post-treatment comparisons of the patient groups with regard to (a) total body calcium retention, (b) radial and spinal bone mineral density as determined by dual-photon absorptiometry (DPA) or dual-energy x-ray absorptiometry (DEXA), (c) bone biopsies of the iliac crest, and (d) determinations of serum osteocalcin. Safety is evaluated by comparisons of urinary hydroxyproline excretion, serum and urine calcium levels, creatinine clearance, blood urea nitrogen, and other routine determinations.

The results show that patients treated with 1α-Vitamin D₂ exhibit significantly higher total body calcium, and radial and spinal bone densities relative to patients treated with placebo. The treated patients also exhibit significant elevations in serum osteocalcin. Bone biopsies obtained from the treated patients show that 1α-Vitamin D₂ stimulates normal bone formation. The monitored safety parameters confirm an insignificant incidence of hypercalcemia or hypercalciuria, or any other metabolic disturbance with 1α-Vitamin D₂ therapy.

### Example 3

A clinical study is conducted with healthy postmenopausal women having ages between 55 and 60 years. The study involves up to 80 patients randomly divided into two treatment groups, and continues for 12 to 18 months. One treatment group receives a constant dosage of 1α-Vitamin D₂ (u.i.d.; a dose level above 3.0 µg/day) and the other receives a matching placebo. The study is conducted as indicated in Example 2 above.

The results show that patients treated with 1α-Vitamin D₂ exhibit reduced losses in total body calcium, radial or spinal bone densities relative to baseline values. In contrast, patients treated with placebo show significant losses in these parameters relative to baseline values. The monitored safety parameters confirm the safety of long-term 1α-Vitamin D₂ administration at this dose level.

### Example 4

A twelve month double-blind placebo-controlled clinical trial is conducted with thirty men and women with renal disease who are undergoing chronic hemodialysis. All patients enter an 8-week control period during which time they receive a maintenance dose of Vitamin D₃ (400 IU/day). After this control period, the patients are randomized into two treatment groups: one group receives a constant dosage of 1α-Vitamin D₂ (u.i.d.; a dosage greater than 3.0 µg/day) and the other group receives a matching placebo. Both treatment groups receive a maintenance dosage of Vitamin D₃, maintain a normal intake of dietary calcium, and refrain from using calcium supplements. Efficacy is evaluated by pre- and post-treatment comparisons of the two patient groups with regard to (a) direct measurements of intestinal calcium absorption, (b) total body calcium retention, (c) radial and spinal bone mineral density, and (d) determinations of serum calcium and osteocalcin. Safety is evaluated by regular monitoring of serum calcium.

Analysis of the clinical data show that 1α-Vitamin D₂ significantly increases serum osteocalcin levels and intestinal calcium absorption, as determined by direct measurement using a double-isotope technique. Patients treated with this compound show normalized serum calcium levels, stable values for total body calcium, and stable radial and spinal bone densities relative to baseline values. In contrast, patients treated with placebo show frequent hypocalcemia, significant reductions in total body calcium and radial and spinal bone density. An insignificant incidence of hypercalcemia is observed in the treated group.

The foregoing examples demonstrate that 1α-Vitamin D₂ is effective in preventing or restoring the loss of bone mass or bone mineral content while being substantially less toxic than 1α-Vitamin D₃. Also included within the scope of the claims would be administration of effective dosages of 1α-Vitamin D₂ in conjunction with administration of other Vitamin D compounds, hormones or other agents which have been shown to stimulate bone formation in subjects experiencing or tending toward loss of bone mass or bone mineral content.

Such other Vitamin D compounds would include Vitamin D, 250H Vitamin D, 1,25 Vitamin D₃, 1α-Vitamin D₃ and chemical variations which retain the characteristics of these Vitamin D compounds and are contemplated as equivalents.

Such hormones or other agents would include conjugated estrogens or their equivalents, calcitonin, biphosphonates, calcium supplements, cobalamin, pertussis toxin and boron. Possible dose ranges for these coadministered agents are provided in Table 2.

**TABLE 2**

| POSSIBLE ORAL DOSE RANGES FOR VARIOUS AGENTS CO-ADMINISTERED WITH 1α-HYDROXYVITAMIN D₂ | | | |
|---|---|---|---|
| AGENT | DOSE RANGES | | |
| | Broad | Preferred | Most Preferred |
| Conjugated Estrogens or Equivalent (mg/day) | 0.3-5.0 | 0.4 - 2.4 | 6.6 - 1.2 |
| Sodium Fluoride (mg/day) | 5 - 150 | 30 - 75 | 40 - 60 |
| Calcitonin (IU/day) | 5 - 800 | 25 - 500 | 50 - 200 |
| Biphosphonates | 50 - 2000 | 100 - 1500 | 250 - 1000 |
| Calcium Supplements (mg/day) | 250 - 2500 | 500 - 1500 | 750 - 1000 |
| Cobalamin (µg/day) | 5 - 200 | 20 -100 | 30 - 50 |
| Pertussis Toxin (mg/day) | 0.1 - 2000 | 10 - 1500 | 100 - 1000 |
| Boron (mg/day) | 0.10 - 3000 | 1 - 250 | 2 - 100 |

Although the examples detail dosage by mouth, it is to be understood that the compounds can be administered in alternative fashions, including intranasally, transdermally, intrarectally, intravaginally, subcutaneously, intravenously, and intramuscularly.

Dosage forms of 1α-Vitamin D₂ can be prepared by dissolving or suspending the compound in, or adsorbing it on, non-toxic pharmaceutically acceptable carriers as is well known in the art. Such carriers may be either solid or liquid such as, for example, corn starch, lactose, sucrose, peanut oil, olive oil, neutral oil, and propylene glycol. If a solid carrier is used, the dosage form of the compound may be tablets, capsules, powders, suppositories, or lozenges. If a liquid carrier is used, soft gelatin capsules, transdermal patches, aerosol sprays, topical creams, syrups or liquid suspensions, emulsions or solutions may be the dosage form. The dosage forms may also contain excipients, such as preserving, stabilizing, wetting, emulsifying agents, etc.

Bulk quantities of 1α-Vitamin D₂ for the practice of this invention can be readily obtained in accordance with the processes of U.S. Patents Nos. 3,907,843, 4,195,027, 4,202,829, 4,234,495, 4,260,549, 4,555,364, and 4,554,106.

## Claims

1. Use of 1-alpha-hydroxy vitamin D₂, for the preparation of a medicament for the treatment or prevention of human osteoporosis or osteopenia by the administration of at least 2.0 µg per day of the 1-alpha-hydroxy vitamin D₂, wherein the medicament is free of parathyroid hormone and free of active fragments thereof.

2. Use of 1-alpha-hydroxy vitamin D₂ for the preparation of a medicament for the reversal of human osteoporosis, by the administration of at least 2.0 µg per day of the 1-alpha-hydroxy vitamin D₂, wherein the medicament is free of parathyroid hormone and free of active fragments thereof.

3. Use according to either claim 1 or claim 2 wherein the medicament also comprises at least one hormone or other agent capable of reducing loss of bone mass or bone mineral content in humans experiencing or tending toward said loss of bone mass or bone mineral content.

4. Use according to claim 3 wherein said hormone or other agent includes vitamin D compounds other than 1-alpha-hydroxy vitamin D₂, conjugated estrogens, calcitonin, sodium fluoride, biphosphonates, calcium supplements, cobalamin, pertussin toxin or boron.

5. Use according to any one of claims 1 to 4 wherein the medicament is suitable to be administered orally.

6. Use according to any one of claims 1 to 4 wherein the medicament is suitable to be administered parenterally.

7. Use according to any one of claims 1 to 4 wherein the medicament is suitable to be administered by subcutaneous, intramuscular, or intravenous injection, nasopharyngeal or mucosal absorption, or transdermal absorption.

8. Use according to any one of claims 1 to 3 wherein the amount of 1-alpha-hydroxy vitamin D₂ in the medicament is sufficient by itself to restore lost bone mass by stimulating bone formation.

9. A pharmaceutical composition which is a single dose in the form of a tablet, lozenge or capsule which contains at least 2.0 µg of 1-alpha-hydroxy vitamin D₂, also contains a pharmaceutically acceptable excipient, and is free of parathyroid hormone and free of active fragments thereof.

10. A pharmaceutical composition as claimed in claim 9 which further comprises at least one hormone or other agent capable of reducing loss of bone mass or bone mineral content in humans experiencing or tending toward said loss of bone mass or bone mineral content.

11. A pharmaceutical composition as claimed in claims 10 wherein said hormone or other agent includes vitamin D compounds other than 1-alpha-hydroxy vitamin D₂, conjugated estrogens, calcitonin, sodium fluoride, biphosphonates, calcium supplements, cobalamin, pertussin toxin or boron.

## Patentansprüche

1. Verwendung von 1-Alphahydroxy-Vitamin D₂ zur Herstellung eines Medikaments zur Behandlung oder Vorbeugung von menschlicher Osteoporose oder Osteopenie durch tägliche Verabreichung von zumindest 2,0 µg 1-Alphahydroxy-Vitamin D₂, worin das Medikament frei von Parathyroidhormon oder aktiven Fragmenten davon ist.

2. Verwendung von 1-Alphahydroxy-Vitamin D₂ zur Herstellung eines Medikaments zur Umkehr von menschlicher Osteoporose durch tägliche Verabreichung von zumindest 2,0 µg 1-Alphahydroxy-Vitamin D₂, worin das Medikament frei von Parathyroidhormon und aktiven Fragmenten davon ist.

3. Verwendung nach Anspruch 1 oder Anspruch 2, worin das Medikament auch zumindest ein Hormon oder einen anderen Wirkstoff enthält, der fähig ist, den Verlust an Knochenmasse oder Knochenmineralstoffgehalt in Menschen zu verringern, die den genannten Verlust an Knochenmasse oder Knochenmineralstoffgehalt aufweisen oder dazu neigen.

4. Verwendung nach Anspruch 3, worin das genannte Hormon oder der andere Wirkstoff andere Vitamin D-Verbindungan als 1-Alphahydroxy-Vitamin D₂, konjugierte Östrogene, Calcitonin, Natriumfluorid, Biphosphonate, Kalziumergänzer, Cobalamin, Pertussintoxin oder Bor umfaßt.

5. Verwendung nach einem der Ansprüche 1 bis 4, worin sich das Medikament zur oralen Verabreichung eignet.

6. Verwendung nach einem der Ansprüche 1 bis 4, worin sich das Medikament zur parenteralen Verabreichung eignet.

7. Verwendung nach einem der Ansprüche 1 bis 4, worin sich das Medikament zur subkutanen, intramuskulären oder intravenösen Injektion, zur nasopharingealen oder mucosalen Aufnahme oder zur transdermalen Aufnahme eignet.

8. Verwendung nach einem der Ansprüche 1 bis 3, worin die Menge an 1-Alphahydroxy-Vitamin D₂ im Medikament als solche ausreichend ist, verlorene Knochenmasse durch Stimulierung der Knochenbildung wiederherzustellen.

9. Pharmazeutische Zusammensetzung, die eine Einzeldosis in Form einer Tablette, Pastille oder Kapsel ist, die zumindest 2,0 µg 1-Alphahydroxy-Vitamin D₂ und auch einen pharmazeutisch verträglichen Exzipienten enthält sowie frei von Parathyroidhormon und aktiven Fragmenten davon ist.

10. Pharmazeutische Zusammensetzung nach Anspruch 9, die weiters zumindest ein Hormon oder einen anderen Wirkstoff enthält, der den Verlust an Knochenmasse oder Knochenmineralstoffgehalt in Menschen verringern kann, die einen Verlust an Knochenmasse oder Knochenmineralstoffgehalt aufweisen oder dazu neigen.

11. Pharmazeutische Zusammensetzung nach Anspruch 10, worin das genannte Hormon oder der Wirkstoff andere Vitamin D-Verbindungan als 1-Alphahydroxy-Vitamin D₂, konjugierte Östrogene, Calcitonin, Natriumfluorid, Biphosphonate, Kalziumergänzer, Cobalamin, Pertussintoxin oder Bor umfaßt.

## Revendications

1. Utilisation de 1-alpha-hydroxy vitamine D₂, pour la préparation d'un médicament pour le traitement ou la prévention de l'ostéoporose humaine ou de l'ostéopénie humaine par l'administration d'au moins 2,0 µg par jour de la 1-alpha-hydroxy vitamine D₂, dans laquelle le médicament est exempt d'hormone parathyroïde et exempt de fragments actifs de celle-ci.

2. Utilisation de 1-alpha-hydroxy vitamine D₂ pour la préparation d'un médicament pour l'inversion de l'ostéoporose humaine, par l'administration d'au moins 2,0 µg par jour de la 1-alpha-hydroxy vitamine D₂, dans laquelle le médicament est exempt d'hormone parathyroïde et exempt de fragments actifs de celle-ci.

3. Utilisation selon la revendication 1 ou 2, dans laquelle le médicament comprend également au moins une hormone ou un autre agent capable de réduire la perte de masse osseuse ou la teneur en minéraux de l'os chez des humains subissant ou tendant vers une perte de masse osseuse ou de teneur en minéraux de l'os.

4. Utilisation selon la revendication 3, dans laquelle ladite hormone ou ledit autre agent inclut des composés de vitamine D autre que la 1-alpha-hydroxy vitamine D₂, conjugués avec des oestrogènes, de la calcitonine, du fluorure de sodium, des biphosphonates, des suppléments de calcium, de la cobalamine, une toxine pertussine ou du bore.

5. Utilisation selon l'une quelconque des revendications 1 à 4, dans laquelle le médicament est approprié pour être administré oralement.

6. Utilisation selon l'une quelconque des revendications 1 à 7, dans laquelle le médicament est approprié pour être administré de façon parentérale.

7. Utilisation selon l'une quelconque des revendications 1 à 4, dans laquelle le médicament est approprié pour être administré par injection souscutanée, intramusculaire, ou intraveineuse, absorption nasopharyngique ou mucosique, ou absorption transdermique.

8. Utilisation selon l'une quelconque des revendications 1 à 3, dans laquelle la quantité de 1-alpha-hydroxy vitamine D₂ dans le médicament est suffisante en elle-même pour restaurer la perte de masse osseuse en simulant la formation d'os.

9. Composition pharmaceutique qui est une dose unique sous la forme d'un comprimé, d'une pastille ou d'une capsule qui contient au moins 2,0 µg de 1-alpha-hydroxy vitamine D₂, et contient également un excipient acceptable pharmaceutiquement, et est exempte d'hormone parathyroïde et exempte de fragments actifs de celle-ci.

10. Composition pharmaceutique selon la revendication 9, qui comprend de plus au moins une hormone ou un autre agent capable de réduire la perte de masse osseuse ou de la teneur en minéraux de l'os chez des humains subissant ou tendant vers une perte de masse osseuse ou de teneur en minéraux de l'os.

11. Composition pharmaceutique selon la revendication 10, où ladite hormone ou ledit autre agent inclut des composés de vitamine D autres que la 1-alpha-hydroxy vitamine D₂, conjugués avec des oestrogènes, de la calcitonine, du fluorure de sodium, des biphosphonates, des suppléments de calcium, de la cobalamine, de la toxine de pertussine ou du bore.
